Europäisches Patentamt

European Patent Office

Office européen des brevets

(19)

(11) Veröffentlichungsnummer: **0 147 851 B1**

# (12) EUROPÄISCHE PATENTSCHRIFT

(45) Veröffentlichungstag der Patentschrift: **11.09.91**

(51) Int. Cl.5: **A61K 31/71**, A61K 47/40

(21) Anmeldenummer: **84116371.0**

(22) Anmeldetag: **27.12.84**

(54) **Wasserlösliche Formen von Polyenantibiotika und Verfahren zu ihrer Herstellung.**

(30) Priorität: **29.12.83 HU 450883**

(43) Veröffentlichungstag der Anmeldung:
**10.07.85 Patentblatt 85/28**

(45) Bekanntmachung des Hinweises auf die
Patenterteilung:
**11.09.91 Patentblatt 91/37**

(84) Benannte Vertragsstaaten:
**CH DE FR IT LI NL**

(56) Entgegenhaltungen:
**EP-A- 0 091 782**
**FR-A- 2 444 060**
**FR-A- 2 515 187**
**GB-A- 978 170**

**CHEMICAL ABSTRACTS, Band 97, 1982, Seite
313, Zusammenfassung Nr. 177021m, Colum-
bus, Ohio, US; & JP-A-82 128 610 (SUMITOMO
CHEMICAL CO. LTD. et al.) 10-08-1982**

(73) Patentinhaber: **CHINOIN Gyogyszer és Vegy-
észeti Termékek Gyára RT.
To utca 1-5
H-1045 Budapest IV(HU)**

(72) Erfinder: **Szejtli, Jozsef, Dr.**

Endrödi S. u. 38-40
**H-1026 Budapest(HU)**
Erfinder: **Vikmon, Maria, Dr. geb. Kiraly**
Endrödi S. u. 24
**H-1026 Budapest(HU)**
Erfinder: **Stadler, Agnes, Dr. geb. Szöke**
Nepstadion u. 18
**H-1143 Budapest(HU)**
Erfinder: **Piukovich, Sandor**
Toth A. sétany 38
**H-1014 Budapest(HU)**
Erfinder: **Inczefi, Istvan**
Rakos ut. 225
**H-1155 Budapest(HU)**
Erfinder: **Kulcsar, Gabor, Dr.**
Szlovak u. 100
**H-1162 Budapest(HU)**
Erfinder: **Jarai, Miklos, Dr.**
Alom u. 20
**H-1125 Budapest(HU)**
Erfinder: **Zlatos, Gabriella, Dr.**
Vasarhelyi K. tér 13
**H-2000 Szentendre(HU)**

(74) Vertreter: **Bartsch, Elisabeth, Dr.
Patentanwälte Lotterhos & Partner Lichten-
steinstrasse 3
W-6000 Frankfurt am Main 1(DE)**

## Beschreibung

Die Erfindung betrifft neue, wasserlösliche und stabile Formen von Polyenantibiotika und ein Verfahren zu ihrer Herstellung.

Dadurch, daß die bakteriellen Infektionen mittels der Antibiotika erfolgreich zurückgedrängt werden konnten, stieg die Zahl der pilzlichen Infektionen, und damit stellte sich die Frage nach Präparaten zur wirksamen Bekämpfung fungaler Infektionen. Zum Teil als Folge der verbreiteten Antibiotikumbehandlungen, zum Teil als Folge der Einnahme oraler Empfängnisverhütungsmittel verbreiten sich die pilzlichen Infektionen immer stärker. Sie sind vor allem in tropischen und subtropischen Ländern ein Problem der Volksgesundheit.

Eine wirksame Bekämpfung der pilzlichen Infektionen wird durch den Umstand erschwert, daß es wesentlich weniger antifungal wirksame Stoffe gibt als antibakterielle Stoffe. Die Polyenantibiotika werden wegen ihrer starken antifungalen Wirkung verbreitet gegen systemische und nicht systemische Mykosen eingesetzt. Bis heute sind in der Literatur etwa hundert Polyenantibiotika beschrieben worden, praktische Bedeutung haben jedoch nur einige.

Für die Struktur der Polyenantibiotika ist charakteristisch, daß ein Teil des Moleküls verhältnismäßig hydrophil ist und zahlreicheHydroxylgruppen enthält, während die andere Hälfte des Moleküls aus mehrfach ungesättigten hydrophoben Chromophorgruppen besteht. Bezeichnend für Polyenantibiotika ist ihre Empfindlichkeit gegen Wärme, UV-Licht, extreme pH-Werte und insbesondere Sauerstoff. Jede Veränderung der Polyenchromophore ist mit dem Verlust der Wirkung verbunden. Mit dem Anstieg der Anzahl der Doppelbindungen wird der hydrophobe Charakter stärker, die Löslichkeit sinkt. Die für die Praxis bedeutsamen Polyenantibiotika sind in Wasser praktisch unlöslich. Bei oraler Verabreichung werden sie kaum oder überhaupt nicht resorbiert, deswegen ist ihre Toxizität per cs vernachlässigbar gering. Bei parenteraler, insbesonders intravenöser Applikation liegt jedoch ihr $LD_{50}$-Wert bei nur wenigen mg/kg Körpergewicht (S.M. Hammond: Biological Activity of Polyene Antibiotics, Progress in Medicinal Chemistry, Vol. 14, North-Holland Publishing Company, 1977, S. 105).

Polyenantibiotika werden auch zum Fernhalten der Pilze von Gewebekulturen, ferner in der Lebensmittelindustrie und der Landwirtschaft gegen pilzliche Schädlinge eingesetzt. Die Anwendung in diesen Richtungen ist jedoch durch die hochgradige Wasserunlöslichkeit der Polyenantibiotika beziehungsweise die Instabilität der aus ihnen bereiteten wäßrigen dispersen Systeme begrenzt.

Die Polyenantibiotika sollten in erster Linie zusammen mit verbreitet angewendeten Fungiziden eingesetzt werden, gegen welche schon in bedeutendem Maße Resistenz entstanden ist. Deshalb muß die für Polyenantibiotika brauchbare Formulierung nicht nur die Wasserlöslichkeit erhöhen und die Stabilität in wäßriger Lösung verbessern, sondern auch die Kompatibilität der Polyenantibiotika mit anderen Wirkstoffen gewährleisten.

Die Polyenantibiotika sind in polaren Lösungsmitteln (z.B. Dimethylsulfoxyd, Dimethylformamid, Äthanol usw.) löslich. Werden diese Lösungen mit Wasser verdünnt, so fällt das Antibiotikum nicht aus, aber es entstehen auch keine echten Lö-sungen, sondern ein aus nicht dialysierbaren Micellen bestehendes instabiles System (J. Kirschbaum: J. Pharm. Sci. 63, 1974, 1981). In derartigen Micellendispersionen treten die hydrophoben Teile der Moleküle miteinander in Kontakt, was für die Polyenchromophore einen gewissen Schutz bedeutet.

Wird die Wasserlöslichkeit erhöht, so sinkt im allgemeinen die chemische Stabilität des Moleküls. Werden zum Beispiel Amphotericin B und sein Methylester unter identischen Bedingungen trocken gelagert, so ist ihre Stabilität gleich. In wäßrigem Medium ist der Methylester des Amphotericins jedoch wesentlich labiler als das Grundmolekül, denn er wird im Wasser besser dispergiert, dadurch ist die Polyenchromophorgruppe weniger geschützt (Bonner: J. Antibiot., 28, 1975, 132).

Mit Natriumdesoxycholat oder Natriumlaurylsulfat (Detergentien) beziehungsweise mit Natriumborat wurden wasserlösliche Komplexe der Polyenantibiotika erhalten; diese ergaben aber in jedem Falle nur kolloidale Dispersionen. Auch durch die Herstellung von Copräzipitaten konnte die Löslichkeit etwas verbessert werden; wird zum Beispiel Nystatin zusammen mit Polyvinylpyrrolidon ausgefällt, so ist seine Löslichkeit 8-10 mal so groß, die Stabilität sinkt jedoch stark ab (M.B. Dexter: J. Pharm. Pharmacol. 27, 1975, S. 58).

Wegen der Wasserunlöslichkeit und der Instabilität in wäßrig-dispersen Systemen war bisher an Anwendungsarten, bei denen stabile wäßrige Lösungen erforderlich sind, nicht zu denken (Injektionslösungen, lokal anwendbare Lösungen wie Augentropfen, Ohrentropfen, oder im Pflanzenschutz versprühbare Lösungen). Bisher sind wäßrige Lösungen von Polyenantibiotika noch nicht hergestellt worden, nur wäßrige Suspensionen, wie zum Beispiel 10-30 000 E/ml fein pulverisiertes Nystatin enthaltende Augentropfen (Martindale: The Extra Pharmacopoeia, The Pharmaceutical Press, London, 1977, S. 649).

EP 0 147 851 B1

Aus der Literatur ist bekannt, daß bisher jede Erhöhung der Wasserlöslichkeit der Polyenantibiotika mit einem Verlust an chemischer Stabilität bezahlt wurde.

Die EP-A 0 091 782 beschreibt $\beta$- und/oder $\gamma$-CD-Komplexe der Lankacidin-Antibiotica.

Aus der FR-A 2 515 187 ist es bekannt, die Wasserlöslichkeit von schlecht wasserlöslichen Steroiden duch Überführen in deren CD-Komplexe zu erhöhen, z.B. um sie in injizierbare Lösungen überführen zu können.

Überraschenderweise wurde nun gefunden, daß die Löslichkeit der Polyenantibiotika bedeutend (auf das 60-80-fache) ansteigt, gleichzeitig ihre chemische und biologische Stabilität jedoch erhalten bleibt, wenn man aus ihnen mit $\gamma$-Cyclodextrin (im folgenden $\gamma$-CD) Komplexe bildet. In der Literatur ist beschrieben, daß die Löslichkeit unterschiedlicher Stoffe mit $\alpha$- und $\beta$-Cyclodextrin gesteigert werden kann (J. Szejtli: Cyclodextrins and their inclusion complexes, Akadémie Kiadó, Budapest, 1982), über $\gamma$-Cyclodextrin sind jedoch nur wenig Angaben zu finden.

Eigene Versuche ergaben, daß weder die Löslichkeit noch die Stabilität der Polyenantibiotika mit $\alpha$- und $\beta$-CD bedeutend erhöht werden können. Von den zahlreichen Polyenantibiotika wurden das Flavofurgin, das Nystatin und das Amphotericin B ausführlicher untersucht.

Das Flavofungin wird von Streptomyces flavofungini produziert. Es ist eine lebhaft gelbe, kristalline Substanz, die bei 210 °C schmilzt und aus einem 10:1 Gemisch von zwei Komponenten besteht, deren Summformel $C_{36}H_{58}O_{10}$ beziehungsweise $C_{37}H_{60}O_{10}$ lautet. Flavofungin verfügt über ein sehr breites antifungales Spektrum. Seine biologische und toxikologische Untersuchung wird dadurch erschwert, daß es in Wasser praktisch unlöslich ist und sich beim Stehen an der Luft zersetzt, seine biologische Wirkung verliert. Steht Flavofungin zum Beispiel bei Raumtemperatur an der Luft, so verliert es innerhalb von zwei Wochen 50 % seiner Aktivität. Unter Stickstoff- oder $CO_2$-Atmosphäre ist es unzersetzt lagerbar. Zu einer praktischen Anwendung kam es deshalb bisher nicht (Uri et al., österreichische Patentschrift Nr. 215 076).

In eigenen Versuchen zur Erhöhung der Löslichkeit und der Stabilität des Flavofungins wurde mit drei verschiedenen Methoden versucht, seinen $\beta$-CD-Komplex herzustellen. Zuerst wurden 121 mg Flavofungin in 1,5 ml Chloroform gelöst, und die Lösung wurde mit Methanol auf 10 ml aufgefüllt. Dann wurden 1,8 $\beta$-Cyclodextrin (Verlust beim Trocknen 13 %) bei 60 °C in 28 ml destilliertem Wasser gelöst. Zu dieser Lösung wurde unter Stickstoff bei ständigem Rühren tropfenweise die vorher bereitete Flavofunginlösung gegeben. Die Lösung wurde zuerst trübe, klärte sich aber nach etwa 5 Minuten völlig. Die Lösung wurde noch eine halbe Stunde lang gerührt, dann langsam abgekühlt, gefroren und lyophilisiert. In dem Produkt lagen $\beta$-CD und Flavofungin in Molverhältnis 1:0,53 vor. Im zweiten Versuch wurde ein Produkt mit dem Molverhältnis 1:0,4 auf die gleiche Weise, aber bei 40 °Chergestellt. Im dritten Versuch wurde das Produkt nicht lyophilisiert, sondern durch 12stündiges Stehen bei 0 °Ckristallisiert. In diesem Produkt waren 7,9 % Flavofungin enthalten.

Die mit den Lösungen der Produkte aufgenommenen UV-Absorptionskurven stimmten mit der des reinen Flavofungins überein. Nach einem Monat Lagerung bei Raumtemperatur und unter Lichtabschluß war eine bedeutende Veränderung des Spektrums zu verzeichnen. Die Extinktion bei 360 nm war abgesunken, die bei 260 nm hingegen angestiegen.

Reines Flavofungin beziehungsweise die auf die beschriebene Weise hergestellten $\beta$-CD-Komplexe wurden bei 32 °C unter reinem Sauerstoff 250 Stunden lang gelagert. Die Sauerstoffaufnahme des komplex gebundenen Wirkstoffes betrug etwa 30 % der Sauerstoffaufnahme des reinen Wirkstoffes. Das $\beta$-CD kann demnach das Flavofungin nur teilweise vor der Oxidation schützen. Auch unter diesen Bedingungen war der Einfluß des Sauerstoffes daran zu sehen, daß das Absorptionsmaximum bei 360 nm völlig verschwand und sich das Bild des ganzen Spektrums änderte.

Der Flavofungin-$\beta$-Cyclodextrin-Komplex erwies sich in mikrobiologischen Versuchen gegenüber Pilzen als völlig wirkungslos.

Es kann demnach festgestellt werden, daß zwischen $\beta$-Cyclodextrin und Flavofungin zwar eine Wechselwirkung eintritt - anders könnte das wasserunlösliche Flavofungin zusammen mit $\beta$-Cyclodextrin keine völlig klare Lösung ergeben - der empfindlichste Teil des Moleküls wahrscheinlich aber nicht vom $\beta$-Cyclodextrin eingeschlossen wird und sich deshalb ein stabiler, biologisch jedoch aktiver Komplex aus Flavofungin und $\beta$-Cyclodextrin nicht herstellen läßt.

Unter dem Aspekt der praktischen Anwendbarkeit konnte zwischen den Polyenantibiotika und $\gamma$-Cyclodextrin eine wesentlich vorteilhaftere Wechselwirkung beobachtet werden.

Der erste Beweis für die Wechselwirkung zwischen Flavofungin und $\gamma$-Cyclodextrin ist der beträchtliche Anstieg der Löslichkeit des Flavofungins in wäßrigen $\gamma$-Cyclodextrinlösungen. Zur Untersuchung der Löslichkeit wurden wäßrige $\gamma$-CD-Lösungen unterschiedlicher Konzentration hergestellt: die Lösungen enthielten 0, 1, 2, 3, 4, 5, 7,5 beziehungsweise 10 % $\gamma$-CD. Zu je 1 ml der Lösungen wurden 5 mg festes Flavofungin gegeben. Die Lösungen wurden bei 25 °C 3 Stunden lang intensiv geschüttelt.

Die Lösungen wurden klarfiltriert, dann wurde spektrophotometrisch die Menge des in Lösung gegange-

3

nen Flavofungins ermittelt. Die Messungen zeigten, daß sich das Flavofungin am besten in 4 % $\gamma$-CD enthaltenden wäßrigen Lösungen löst; unter den Bedingungen der Untersuchung können Konzentrationen von etwa 4 mg/ml erzielt werden. Da die Löslichkeit des Flavofungins in Wasser bei 0,05 mg/ml liegt, entspricht das einem Anstieg der Löslichkeit auf etwa das Achtzigfache. Eine weitere Erhöhung der $\gamma$-CD-Konzentration steigert die Löslichkeit des Flavofungins nicht weiter. Die Ergebnisse der Löslichkeitsuntersuchungen sind in der Tabelle 1 zusammengefaßt.

### Tabelle 1

### Löslichkeit des Flavofungins als Funktion der $\gamma$-CD-Konzentration (25 °C, 3 Stunden Schütteln)

| $\gamma$-CD-Konz., % | Flavofungin-Konz., mg/ml |
| --- | --- |
| 0 | 0,05 |
| 1 | 1,0 |
| 2 | 1,8 |
| 3 | 3,0 |
| 4 | 4,0-4,2 |
| 5 | 3,8 |
| 7,5 | 3,7 |
| 10 | 3,8 |

Die aus dem anfänglich linearen Abschnitt der bei 25 °C aufgenommenen Löslichkeitsisotherme berechnete scheinbare Stabilitätskonstante des Komplexes beträgt etwa 3230.

Die maximale Löslichkeit in 4 %iger $\gamma$-CD-Lösung wurde auch bei verschiedenen Temperaturen untersucht. Die Flavofunginkonzentration betrug bei 10 °C 3,3 mg/ml, bei 30 °C 4,1 mg/ml und bei 40 °C 3,8 mg/ml.

Das Nystatin wird durch Fermentation von Streptomyces noursei, S. albulus oder S. aureus hergestellt. Es ist das in der größten Menge erzeugte Polyenmakrolidantibiotikum. Das im Handel erhältliche Nystatin ist keine einheitliche Substanz, sondern ein Gemisch chemisch nahe verwandter, mit $A_1$, $A_2$ und $A_3$ bezeichneter Komponenten (Sherin et al.: Antibiotiki 13, 987 [1968]). Eben deshalb sind Isolierung und befriedigende analytische Kennzeichnung der Substanz schwierig; abhängend von Ursprung, Reinheit und Einheitlichkeit erhält man eine breite Skala physikalischer und chemischer Eigenschaften.

Nystatin ist bei Raumtemperatur in Wasser und unpolaren Lösungsmitteln praktisch unlöslich, löst sich jedoch gut in Formamid, Dimethylformamid, Dimethylsulfoxyd, Pyridin, Äthylenglycol und Propylenglycol. Die Löslichkeit in polaren Lösungsmitteln steigt in Gegenwart von 10-20 % Wasser bedeutend an. Zum Beispiel lösen sich in 75 %-igem wäßrigem Äthanol 4 mg/ml (in reinem Äthanol 0,55 mg/ml), in 70 %igem wäßrigem Isopropanol 2,2 mg/ml (in reinem Isopropanol 0,62 mg/ml) (Trakhtenberg et al.: Antibiotiki 5, 9 [1960]; Analytical profiles of drug substances Vol. 6. Academic Press, New York and London 1977, p. 344). Die maximale Löslichkeit in Methanol beträgt 9,2-11,2 mg/ml (Weiss et al.: Antibiot. Chemotherapy 7, 374 [1957]). Nystatin wird meistens topisch gegen Monilia-Infektionen der Haut, der Nägel und der Schleimhäute eingesetzt. Zur Heilung der Candidiase des Magen-Darm-Traktes wird es auch per os appliziert. Gegen systemische Mykosen ist es jedoch wirkungslos, da das per os verabreichte Nystatin nicht resorbiert wird (sein Blutspiegel ist überhaupt nicht meßbar).

Ähnlich wie im Falle des Flavofungins wurde auch für Nystatin die Löslichkeit in $\gamma$-CD-Lösungen unterschiedlicher Konzentration ermittelt. Die verwendeten wäßrigen $\gamma$-CD-Lösungen hatten Konzentrationen von 0, 1, 2,5, 4, 5, 6, 7, 8, 9, 10 und 15 %. Pro ml wurden 20 mg Nystatin zu gesetzt. Die Lösungen wurden bei 37 °C 10 Minuten lang geschüttelt, dann wurde ihr Nystatingehalt spektrophotometrisch bestimmt. Ähnlich wie bei Flavofungin wurde gefunden, daß die Löslichkeit des Nystatins in wäßrigen $\gamma$-CD-Lösungen ansteigt und in 5 %iger $\gamma$-CD-Lösung etwa das 25fache, in 10 %iger $\gamma$-CD-Lösung etwa das 40fache der ursprünglichen Wasserlöslichkeit beträgt. Die Meßergebnisse sind in der Tabelle 2 zusammengestellt.

## Tabelle 2

### Löslichkeit des Nystatins (biologische Aktivität 5033 E/mg) als Funktion der $\Gamma$-CD-Konzentration (37 °C, 10 min schütteln)

| $\Gamma$-CD-Konzentration , % | Nystatinkonzentration mg/ml |
|---|---|
| 0 | 0,20 |
| 1 | 1,30 |
| 2,5 | 2,36 |
| 4 | 3,95 |
| 5 | 4,70 |
| 6 | 5,50 |
| 7 | 6,30 |
| 8 | 7,20 |
| 9 | 8,10 |
| 10 | 8,95 |
| 15 | 8,20 |

Die aus dem anfänglich linearen Abschnitt der bei 37 °C aufgenommen Löslichkeitsisotherme berechnete scheinbare Stabilitätskonstante des Komplexes beträgt etwa 500.

Amphotericin B ist ein von Streptomyces nodosus produziertes Polyenantibiotikum. Sein Wirkungsspektrum ist breit, außer auf Hefen und Pilze wirkt es noch gegen zahlreiche Protozoen. Es wird am verbreitetsten zur Behandlung akuter systemischer Pilzinfektionen angewendet. Es kann oral und als mehrstündige intravenöse Infusion appliziert werden. Für letzteren Zweck wird es in Form seines Natriumdesoxycholat-Komplexes verwendet (Markenname: Fungizone).

Die intravenöse Applikation von mit den Salzen der Gallensäure gebildeten Komplexen kann jedoch Brechreiz, Anämie und Nierenschäden verursachen, weil diese Komplexe - zwar ist ihre Löslichkeit gut - viel toxischer sind als andere Derivate. Die Toxizität des Methylesters von Amphotericin B (i.p. an Mäusen) beträgt zum Beispiel $LD_{50}$ = 1320 mg/kg, die auf die gleiche Weise gemessene Toxizität des mit Natriumdesoxycholat gebildeten Komplexes liegt bei $LD_{50}$ = 280 mg/kg. Die größere Toxizität des Desoxycholat-Komplexes wird mit großer Wahrscheinlichkeit durch die Gegenwart des Salzes der Gallensäure verursacht (Thomas: The Analyst 101, 321 [1976]).

Bei oraler Applikation sind niedrige Blutspiegel meßbar, d.h. das Antibiotikum ist zur per os erfolgenden Behandlung systemischer Mykosen geeignet. Die zur parenteralen Anwendung empfohlenen, Amphotericin B enthaltenden Präparate sind jedoch keine echten Lösungen, wie auch die Konzentrationsabhängigkeit des UV-Spektrums zeigt. Das charakteristische UV-Spektrum des Amphotericin B, das der Heptaenchromophor-Gruppe zuzuschreiben ist, ändert sich in wäßriger Lösung. Das Spektrum zeigt Absorptionsmaxima bei 408, 385, 366 und 347 nm, deren Peakhöhe mit sinkender Wellenlänge abnimmt; des ist charakteristisch für das Monomer in einer Konzentraion von etwa 4 μg/ml. Bei höheren Konzentrationen verschwinden die Maxima zwischen 347-408 nm allmählich, während bei 325 nm ein neues Absorptionsmaximum erscheint und mit dem Anstieg der Konzentration intensiver wird. Das gleiche spektrale Verhalten kann an Fungizone beobachtet werden, welches ebenfalls ein kolloid-disperses System darstellt. Der Zusatz von Salz zu derartigen Lösungen verringert die Löslichkeit und fördert die Aggregation, weil es die elektrostatische Abstoßung zwischen den Molekülen vermindert.

Die wäßrige Lösung von Amphotericin folgt dem Gesetz von Beer und Lambert nicht, was ebenfalls ein Kennzeichen des Vorhandenseins von Aggregaten in wäßriger Lösung ist. In mehr als 50 % Alkohol enthaltenden Lösungen stimmt das Spektrum schon gut mit dem Spektrum des Monomers überein, d.h. die Aggregate zerfallen (Rinnert et al.: Biopolymers, 616, 2419-2477 [1977]).

Das UV-Spektrum von Amphotericin B und γ-CD enthaltenden wäßrigen beziehungsweise mit Phosphatpuffer pH 7,5 bereiteten Lösungen stimmt mit dem in organischen Lösungsmitteln meßbaren Spektrum überein, d.h. es zeigt die für das Monomer charakteristischen Absorptionsmaxima bei 408, 385, 366 und 347 nm und folgt dem Gesetz von Beer und Lambert. Dieses Spektralverhalten der mit γ-Cyclodextrin bereiteten Lösungen sind ein Beweis dafür, daß in ihnen das Amphotericin molekular dispers, als echte Lösung vorliegt; das konnte bisher mit keinem Komplex des Amphotericin B erreicht werden, denn diese Komplexe, zum Beispiel der mit Borat beziehungsweise mit Natriumdesoxycholat gebildete Komplex, liegen in wäßrigem Medium als Micellendispersionen vor.

Die Löslichkeit des Amphotericin B sinkt in physiologischen Salzlösungen in jedem Falle, was der Anwendung als Infusion Grenzen setzt. Zum Beispiel beträgt die Löslichkeit des Boratkomplexes von Amphotericin in Wasser 0,54 mg/ml, aber in isotonischer Kochsalzlösung sinkt dieser Wert auf 0,02 mg/ml (Kral et al.: J. of Antibiotics 31(3) 257-259 [1978]).

Demgegenüber wird die Löslichkeit des Amphotericin B in γ-CD-haltigen Lösungen durch den Zusatz von 0,9 Gew.-% Kochsalz nicht verändert, sondern behält genau den Wert, der in der Tabelle 3 angegeben ist. Das ist ein weiterer Beweis dafür, daß das Amphotericin in den γ-CD-haltigen wäßrigen Lösungen in molekular disperser Form vorliegt.

Auch für Amphotericin B wurde die Löslichkeit als Funktion der γ-CD-Konzentration ermittelt. Zu wäßrigen γ-CD-Lösungen unterschiedlicher Konzentration (0, 2, 4, 5, 6, 8, 10 beziehungsweise 12 %) wurden 5 mg/ml Amphotericin B gegeben, und die bei 37 °C thermostatisierten Lösungen wurden 2 Stunden lang intensiv geschüttelt. Die Lösungen wurden klarfiltriert, und die Menge des in Lösung gegangenen Amphotericin B wurde spektrophotometrisch bestimmt. Die Ergebnisse sind in der Tabelle 3 zusammengestellt.

## Tabelle 3

### Die Löslichkeit von Amphotericin B als Funktion der γ-CD-Konzentration

| γ-CD-Konzentration (%) | Amphotericin B Konzentration, mg/ml |
|---|---|
| 0 | 0,003 |
| 2 | 0,12 |
| 4 | 0,25 |
| 5 | 0,30 |
| 6 | 0,36 |
| 8 | 0,51 |
| 10 | 0,65 |
| 12 | 0,65 |

Es ist ersichtlich, daß die Löslichkeit von Amphotericin B in 5 %iger γ-CD-Lösung etwa das Hundertfache, in 10 %iger γ-CD-Lösung etwa das Zweihundertfache der Löslichkeit in Wasser beträgt.

Die Assoziationsverhältnisse in wäßrigen Lösungen von Amphotericin B können besonders vorteilhaft mit der Methode der Zirkulardichroismus-Spektroskopie untersucht werden, weil das Monomer, das Dimere und die Polymeren höheren Assoziationsgrades gut unterscheidbare Zirkulardichroismus-Spektren geben. Diese Spektren wurden für den 0,6 % Wirkstoff enthaltenden Komplex aus Amphotericin und γ-CD in wäßriger Lösung, für Amphotericin B in 50 %iger äthanolischer Lösung untersucht.

Wie aus der Literatur bekannt, bildet Amphotericin B in 50 %igem Äthanol eine echte Lösung, in der die monomeren Moleküle getrennt vorliegen (Ernst et al., Biopolymers 20, 1575 [1981]). Durch Messungen konnte bestätigt werden, daß das in Wasser aufgenommene Spektrum des Komplexes dem in 50 %igem Alkohol aufgenommenen Spektrum des Amphotericins ähnlich ist. Im Spektrum der wäßrigen Lösung des Komplexes erscheinen die für monomeres Amphotericin B charakteristischen Banden, und zwar eine aus

EP 0 147 851 B1

vier Schwingungskomponenten zusammengesetzte positive Bande zwischen 420 und 340 nm und eine aus drei Komponenten bestehende negative Bande zwischen 290 und 240 nm. Zwischen 330 und 300 nm gibt das Monomer kein Spektrum.

Auf die erwähnte Weise konnte festgestellt werden, daß in der wäßrigen Lösung des aus Amphotericin B und γ-CD bestehenden Komplexes bei ausreichend großen Konzentrationen an γ-CD das Amphotericin B ebenso in monomerer Form stabilisiert wird wie in 50 %iger äthanolischer Lösung. In verdünnteren Lösungen dissoziiert der Komplex, doch das freigesetzte Amphotericin wird nur dimerisiert, denn das gegenwärtige γ-CD verhindert die Ausbildung Polymerer mit höherem Assoziationsgrad.

Zum Gegenstand der Erfindung gehört auch ein Verfahren zur Herstellung der neuen, wasserlöslichen und stabilen Polyenantibiotikum-Derivate. Diese werden erfindungsgemäß hergestellt, indem man in Wasser beschränkt lösliche beziehungsweise praktisch unlösliche Polyenantibiotika in Gegenwart von Wasser unter Rühren mit γ-CD umsetzt. Auf 1 mMol Antibiotikum rechnet man 1-40 mMol γ-CD und 1-500 ml Wasser. Das Verfahren kann auf zweierlei Weise ausgeführt werden.

a) Das Polyenantibiotikum wird auf 1 mMol Antibiotikum bezogen in 20-500 ml Wasser mit 1-40 mMol γ-CD unter Rühren bei 25-55 °C umgesetzt, und der erhaltene Antibiotikum-γ-CD-Komplex wird gewünschtenfalls isoliert, oder

b) das Polyenantibiotikum wird auf 1 mMol Antibiotikum bezogen in 1-50 ml eines 20-80 vol.-%igen, wassermischbaren Lösungsmittels, vorzugsweise Äthanol, unter Rühren beziehungsweise Kneten mit 1-40 mMol γ-CD umgesetzt, und der erhaltene Antibiotikum-γ-CD-Komplex wird durch Lyophilisieren, Zerstäubungstrocknung, Vakuumeindampfen oder Vakuumtrocknung bei niedriger Temperatur isoliert.

Die erfindungsgemäßen Antibiotikum-γ-CD-Komplexe können zur Herstellung stabiler injizierbarer und lokal anwendbarer Arzneimittelpräparate beziehungsweise zur Herstellung von Pflanzenschutzmittelkombinationen verwendet werden.

Zum Gegenstand der Erfindung gehören ferner Arzneimittelpräparate mit antifungaler Wirkung, vorzugsweise Puder, Augen- und Ohrentropfen, Salben, Tabletten, Scheidenzäpfchen, Injektionslösungen, zum Schutz von Gewebekulturen vor Pilzbefall geeignete Zusatzstoffe, die als Wirkstoff 1-80 Gew.-% Komplexe aus Polyenantibiotika und γ-CD sowie die für pharmazeutische Produkte üblichen Streck- und Hilfsstoffe enthalten.

Zum Gegenstand der Erfindung gehören auch fungizid wirksame Pflanzenschutzmittel, vorzugsweise Saatgutbeizmittel und Sprühmittel, die als Wirkstoff 0,25-30 Gew.-% Polyenantibiotikum-γ-CD-Komplexe und die bei der Herstellung von Pflanzenschutzmitteln üblichen Streck-, Träger- und/oder Hilfsstoffe enthalten, sowie Zusatzstoffe mit antifungaler Wirkung für Gewebekulturen zur Vermeidung von Pilzbefall, gekennzeichnet durch einen Gehalt an 0,025-5 mg/ml Polyenantibiotikum-γ-Cyclodextrin-Komplex.

Aus den erfindungsgemäßen Polyenantibiotikum-γ-CD-Komplexen können homogene Lösungen bereitet werden. So kann zum Beispiel das aus der Literatur bekannte Augentropfenpräparat, welches 10-30 000 E/ml Nystatin in Form einer wäßrigen Suspension enthält, durch Auflösen von 30-70 mg/ml Nystatin-γ-CD-Komplex als homogene Lösung hergestellt werden. Die erhöhte Wasserlöslichkeit ist bei lokal applizierbaren Mitteln wahrscheinlich mit einer erhöhten Wirksamkeit verbunden.

Hinsichtlich der Komplexbildung verhalten sich alle Polyenantibiotika gleich. Außer den beschriebenen Antibiotika können demnach auch zum Beispiel Candicidin, Pimaricin, Candidin, Hamycin, Candihexin usw. verwendet werden. Deshalb ist die Erfindung nicht auf die beschriebenen Antibiotika beschränkt.

Die Einzelheiten der Erfindung werden im folgenden an Hand einiger die Herstellung der Antibiotikum-γ-CD-Komplexe sowie die Anwendung und Formulierung der Komplexe betreffender Beispiele näher erläutert.

Beispiel 1

Herstellung des Flavofungin-γ-Cyclodextrin-Komplexes in homogener Phase

22 g γ-Cyclodextrin (Wassergehalt 1,5 %) werden bei 30 °C in 500 ml destilliertem Wasser gelöst und zu der Lösung 2,5 g festes Flavofungin gegeben. Die Lösung wird 3 Stunden lang intensiv gerührt, dann filtriert und das erhaltene, intensiv gelbe Filtrat lyophilisiert. Man erhält 21 g Produkt, dessen spektrophotometrisch gemessener Flavofungingehalt 9,3 % beträgt. Das entspricht etwa 80 % der eingesetzten Flavofunginmenge. Der Komplex ist ein lockeres, gelbes Pulver. 100 mg Komplex lösen sich in 2,5 ml destilliertem Wasser bei 50 °C innerhalb von Augenblicken zu einer klaren Lösung. Diese Lösung enthält 3,6 mg/ml Flavofungin und erleidet, 6 Tage lang bei Raumtemperatur und gestreutem Licht gelagert, keinerlei auf chemischem oder physikalischem Wege nachweisbare Veränderungen.

Aus der Tabelle 1 ist ersichtlich, daß die maximale Löslichkeit bei γ-CD-Gehalten von etwa 4 % auftritt

7

(Flavofunginkonzentration 4 mg/ml), deshalb wird durch Lyophilisieren dieser Lösung der Komplex mit der vorteilhaftesten Zusammensetzung (9-10 % Wirkstoff) erhalten.

Thermogravimetrische Messungen, Untersuchungen mit dem Differential-scanning-Kalorimeter (DSC) und die Thermal Evolution Analyse (TEA) zeigen zwischen Flavofungin, Flavofungin-$\gamma$-CD-Komplex und Gemischen aus Flavofungin und $\gamma$-CD einen charakteristischen Unterschied. Das Flavofungin verliert bis 100 °C 7 % seiner Masse (auf Grund der TEA-Kurve treten organische Stoffe aus), beginnt oberhalb von 100 °C sich zu zersetzen und büßt bis 300 °C 24 % seiner Masse ein. Die lyophilisierte Probe aus Flavofungin und $\gamma$-CD erweist sich einesteils dadurch als Komplex, daß bis 100 °C keine organischen Stoffe entweichen, zum anderen, daß in der DSC-Kurve zwischen 100 und 200 °C die für die Oxydation des Flavofungins charakteristische exotherme Veränderung nicht eintritt, dafür aber bei 263 °C ein für exotherme Prozesse charakteristisches Peak erscheint, das die Oxydation des aus dem Komplex freigesetzten Flavofungins anzeigt.

Zur Untersuchung der Wärmestabilität des Flavofungin-$\gamma$-CD-Komplexes wurden die Substanzen in 1 mm Schichtdicke auf Uhrgläsern ausgebreitet und weder vor Licht noch vor Luft geschützt bei Raumtemperatur beziehungsweise bei 60 °C im Trockenschrank gelagert. Die zu unterschiedlichen Zeitpunkten genommenen Proben wurden spektrophotometrisch analysiert.

Auf Grund chemischer Messungen wurde gefunden, daß das nicht in einen Komplex eingeschlossene Flavofungin bereits nach 24 Stunden bei 60 °C starke Zersetzung erlitt (das wurde auch durch Aufnahme des vollständigen UV-Spektrums bestätigt). Der Wirkstoffgehalt des Flavofungin-$\gamma$-CD-Komplexes blieb jedoch auch nach 50 Tagen zu etwa 75 % erhalten. Ein 10 % Flavofungin enthaltendes, mit Glucose bereitetes physikalisches Gemisch verlor bei 14tägiger Lagerung bei Raumtemperatur 50 % seines Wirkstoffgehaltes. Der Wirkstoffgehalt des Komplexes war jedoch auch nach 50 Tagen noch zu mehr als 95 % erhalten. Die in einem verschlossenen Gefäß bei +4 °C 7 Monate lang gelagerte Probe zeigte keinen Verlust an Wirkstoff. Die Meßergebnisse sind in der Tabelle 4 zusammengestellt.

### Tabelle 4

Wärmestabilität bei Raumtemperatur und bei 60 °C, ausgedrückt in Prozent des ursprünglichen Wirkstoffgehaltes

| Zeit (Tage) | bei 60 °C | | bei Raumtemperatur | |
|---|---|---|---|---|
| | Flavofungin | Flavofungin-$\gamma$-CD-Kompl. | Gemisch aus Flavofungin und Glucose | Flavofungin-$\gamma$-CD-Kompl. |
| | verbliebener Wirkstoff, % | | | |
| 0 | 100 | 100 | 100 | 100 |
| 1 | 43,8 | | | |
| 2 | 23 | 100 | 90 | 100 |
| 5 | | 99,5 | 81 | 99,5 |
| 7 | | 99 | | 99 |
| 10 | | 98 | 65 | 96 |
| 15 | | 94 | 49 | 94,8 |
| 20 | | 85,4 | | 95,0 |
| 29 | | 70,5 | | 95,0 |
| 35 | | 79,2 | | 94,9 |
| 44 | | 78,7 | | 94,8 |
| 50 | | 75,0 | | 94,5 |

Auch die mikrobiologische Wertmessung bestätigte, daß das Flavofungin in Form des γ-CD-Komplexes viel stabiler ist. Flavofungin-γ-CD-Komplex wurde bei Raumtemperatur und bei 60° C gelagert, und danach war die antifungale Wirkung genau so groß wie ursprünglich. (MIC-Wert der unter Stickstoff im Tiefkühlfach gelagerten Flavofunginprobe MIC = 25 μg/ml, untersucht an den Testmikroorganismen S. oerevisiae, C. albicans, C. crusei und C. pseudotropicals).

Beispiel 2

Herstellung des Flavofungin-γ-CD-Komplexes in heterogener Phase

9 g γ-Cyclodextrin (Wassergehalt 1,5 %) werden in der Reibschale mit 1 g Flavofungin homogenisiert. Das Gemisch wird in 5 ml 50 %igem Äthanol suspendiert. Die Suspension wird nach einigen Minuten Rühren plötzlich fest. Die pastenartige Substanz wird zu einer dünnen Schicht breitgestrichen und im Vakuumexsikkator einige Stunden lang getrocknet. Dann wird der feste Komplex pulverisiert. 10 g Produkt mit 10 Gew.-% Wirkstoff liegen vor.

Auch mit diesem Produkt wird die im Beispiel 1 beschriebene Untersuchung der Wärmestabilität vorgenommen. Dabei wurde gefunden, daß der Komplex, ähnlich wie der lyophilisierte Komplex, eine erhöhte Wärmestabilität zeigt und seine antifungale Wirkung nicht geringer wird.

Auf den Röntgendiffraktionsaufnahmen des pulverisierten Komplexes erscheinen die charakteristischen Reflexionspeaks bei einem signifikant abweichenden 2 Ø° Winkelwert, an anderer Stelle als im Falle eines physikalischen Gemisches ähnlicher Zusammensetzung. Das läßt auf ein anderes Kristallgitter schliessen und stützt damit die Tatsache der Komplexbildung. Die Röntgendiffraktionsaufnahme des lyophilisierten Flavofungin-γ-CD-Komplexes weist auf eine amorphe Struktur hin.

Beispiel 3

Herstellung des Nystatin-γ-CD-Komplexes in homogener Phase

10 g γ-CD (Wassergehalt 1,5 %) werden bei 50 °C in 90 ml destilliertem Wasser gelöst. Zu der Lösung werden 2 g Nystatin (biologische Aktivität 5033 E/mg) gegeben. Die Lösung wird 15 Minuten lang intensiv gerührt. Nach dem Filtrieren wird die gelbe Lösung lyophilisiert. Man erhält 10 g Produkt, dessen spektrophotometrisch bestimmter Nystatingehalt 8 % beträgt. Biologische Aktivität: 399 E/mg (die 2. internationale Einheit des Nystatin ist 4855 IU/mg, wobei IU der Aktivität von 0,0002059 mg entspricht; Thomas-Dixon: J. Biol.-Stand, 10 (4), 369-377 [1982]). Der Nystatin-γ-CD-Komplex ist ein hellgelbes lockeres Pulver, das sich in Wasser bei Raumtemperatur schnell zu einer klaren Lösung löst. In der Lösung ist eine Konzentration von 8-9 mg/ml Nystatin meßbar, was etwa 100 mg Komplex/ml Lösung entspricht. Die biologische Wertbestimmung des Komplexes ergab, daß die biologische Aktivität des Ausgangsn statin erhalten geblieben war.

Thermogravimetrische Messungen, Untersuchungen mit dem Differential-scanning-Kalorimeter (DSC) und die Thermal Evolution Analyse (TEA) zeigen zwischen Nystatin, Nystatin-γ-CD-Komplex und Nystatin-γ-CD-Gemisch einen charakteristischen Unterschied. Nystatin enthält 3 % Wasser, welches bis 100 °C abgegeben wird ($T^G$-Kurve). Die Zersetzung der Substanz beginnt bei 150 °C mit einem stark exothermen Prozeß (in der DSC-Kurve Peak bei 165 °C) unter 8% Gewichtsverlust (TG-Kurve), daran schließt sich langsame Zersetzung an, und bei 350 °C ist ein Masseverlust von 54 % meßbar. Obwohl die Zersetzung des Nystatins und des γ-CD hinsichtlich der Temperatur zusammenfallen, sind doch in den thermoanalytischen Kurven des Komplexes beziehungsweise des Gemisches einige Abweichungen zu finden, die im Lyophilisat das Vorliegen eines Komplexes wahrscheinlich machen. In der TEA-Kurve fehlt der scharfe Peak bei 160 °C, der auf Entweichen organischer Substanz hinweist. In der DSC-Kurve erscheint der für die Zersetzung des Nystatin charakteristische exotherme Peak bei 165 °C nicht, exotherme Prozesse des Komplexes setzen erst oberhalb von 200 °C ein. In der TG-Kurve manifestiert sich der Unterschied darin, daß sich die lyophilisierte Probe oberhalb von 250 °C schneller zersetzt als das physikalische Gemisch. Die Komplexbildung des Nystatin mit γ-CD wird auch durch $^{13}$C-NMR-Untersuchungen gestützt.

Die Stabilität der aus der lyophilisierten Probe des Nystatin-γ-CD-Komplexes bereiteten Lösungen wurde folgendermaßen untersucht: aus dem 8 % Wirkstoff enthaltenden Komplex wurde eine wäßrige Lösung der Konzentration 5 mg/ml bereitet. Die Lösung wurde bei Raumtemperatur, nicht vor Licht geschützt gelagert. Zu unterschiedlichen Zeitpunkten wurde der Wirkstoffgehalt dar Lösungen spektrophotometrisch kontrolliert.

Tabelle 5

| Zeit (Tage) | Nystatin-Konzentration (mg/ml) |
|---|---|
| o | 5,0 |
| 1 | 4,8 |
| 2 | 4,75 |
| 5 | 4,70 |
| 6 | 4,6 |
| 7 | 4,55 |
| 8 | 4,5 |
| 10 | 4,5 |
| 12 | 4,5 |
| 21 | 4,1 |

Die Lösungen begannen am zweiten Tag zu opalisieren. Die Probenahme erfolgte nach dem Aufschütteln der Lösung. Die Probe wurde mit der 400-600fachen Menge Äthylalkohols verdünnt und dann photometriert. Die Berechnung erfolgte aus den bei $\lambda_{max}$ = 304±1 erscheinenden Extinktionswerten. Aus der Tabelle 5 ist ersichtlich, daß auch nach 3 Wochen Lagerung der wäßrigen Lösung noch über 80 % des Wirkstoffgehaltes vorhanden sind.

Darüber, inwieweit die biologische Aktivität mit den spektrophotometrisch gemessenen Werten in Zusammenhang steht, sind in der Literatur widersprüchliche Angaben zu finden. Gemäß manchen Autoren verläuft der Verlust der biologischen Aktivität vier- bis achtmal so schnell, als sich aus dem Abnehmen der Extinktion folgern ließe, andere Autoren wiederum sind der Meinung, daß Aktivität und Extinktion parallel absinken (Hamilton-Miller: J. Pharm. Pharmac. 25, 401 [1973]). Gemäß eigenen Erfahrungen gehören zu hohen Extinktionswerten hohe Aktivitätswerte.

Beispiel 4

Herstellung von Nystatin-γ-Cyclodextrin-Komplex in heterogener Phase

9 g γ-Cyclodextrin (Wassergehalt 1,5 % und 1 g Nystatin (biologische Aktivität 5033 E/mg) werden in einer Reibschale miteinander homogenisiert. Das erhaltene Gemisch wird in 6 ml 50 %igem Äthanol suspendiert. Nach einigen Minuten wird die Suspension plötzlich fest. Die Substanz wird zerkleinert, ausgebreitet und zur Entfernung der Wasser- und Lösungsmittelspuren im Exsikkator über Phosphorpentoxyd getrocknet. Am nächsten Tag wird der feste Komplex pulverisiert. Man erhält 10 g Produkt mit einem Wirkstoffgehalt von 10 Gew.-% (biologische Aktivität: 450 E/mg).

Der auf die beschriebene Weise hergestellte Komplex zeigte bei den thermoanalytischen Untersuchungen im Vergleich zum Gemisch die gleichen Abweichungen wie der gemäß Beispiel 3 durch Lyophilisieren hergestellte Komplex. Die Löslichkeit von einer der Wirkstoffmenge von 10 mg/ml entsprechenden Menge Nystatin-γ-CD-Komplex und von Nystatingrundmaterial wurde in 15 ml destilliertem Wasser bei 37 °C untersucht. Die Lösungen wurden mit dem Magnetrührer bei einer Drehzahl von 150 min$^{-1}$ gerührt. Zu unterschiedlichen Zeitpunkten wurden Proben genommen, filtriert und spektrophotometrisch auf ihren Wirkstoffgehalt untersucht. Die Ergebnisse sind in der Tabelle 6 zusammengestellt.

EP 0 147 851 B1

## Tabelle 6

| Zeit (min) | Nystatin-γ-CD-Komplex (mg/ml) |
|---|---|
| 2 | 9,0 |
| 5 | 7,2 |
| 15 | 7,8 |
| 40 | 7,4 |
| 60 | 7,2 |
| 90 | 6,0 |

Das Spektrum der von der wäßrigen Suspension des Nystatingrundmaterials genommenen Proben war nicht auswertbar. Das Spektrum der innerhalb der ersten 5 Minuten genommenen Probe war mit dem Spektrum des Nystatin identisch, später war fortschreitende Zersetzung zu beobachten. Zuerst deformierte sich das Maximum um 290 nm und verschwand dann ganz, und im Bereich geringerer Wellenlänge zeigten sich neue Maxima, was auf Schädigung des konjugierten Chromophorsystems schließen läßt.

Beispiel 5

Herstellung von Nystatin-γ-CD-Komplex in heterogener Phase durch Lyophilisieren

8 g γ-CD (Wassergehalt 1,5 %) werden bei 40 °C in 80 ml Wasser gelöst und zu der Lösung 2 g Nystatin (biologische Aktivität: 5033 E/mg) gegeben. Die Suspension wird bei 40 °C 24 Stunden lang gerührt, dann wird das Gemisch durch Lyophilisieren wasserfrei gemacht. In Form eines gelben, lockeren Pulvers erhält man 10 g Produkt, dessen spektrophotometrisch gemessener Wirkstoffgehalt 20 % beträgt. Biologische Aktivität: 950 E/mg.

In dem Produkt beträgt das Molverhältnis zwischen Nystatin und γ-CD 1:3. Nach röntgendiffraktometrischen Untersuchungen hat der Komplex eine amorphe Struktur. Die auf die gleiche Weise, aber ohne γ-CD behandelte Grundsubstanz behält ihren kristallinen Charakter, ihr Röntgendiffraktogramm ist völlig mit dem der Grundsubstanz identisch, ihre biologische Aktivität ist jedoch wesentlich schlechter. Wird auf die beschriebene Weise ein Komplex mit höherem Wirkstoffgehalt hergestellt, so zeigt das Röntgendiffraktogramm bereits die für kristallines Nistatin charakteristischen Peaks.

Zur Untersuchung der Löslichkeit des Komplexes werden 500 mg in 10 ml Wasser 5 Minuten lang bei Raumtemperatur gerührt. Im Filtrat ist spektrophotometrisch eine Nistatinkonzentration von 4,5 mg/ml nachweisbar.

Beispiel 6

Herstellung des Komplexes aus Amphotericin B und γ-CD

10 g γ-Cyclodextrin (Wassergehalt: 1,5 %) werden in 90 ml destilliertem Wasser bei 37 °C gelöst. Zu der Lösung werden 200 mg Amphotericin B gegeben. Das Gemisch wird 2 Stunden lang intensiv gerührt, dann filtriert und das gelbe Filtrat lyophilisiert. Man erhält 10 g Produkt, dessen spektrophotometrisch gemessener Gehalt an Amphotericin B 0,6-0,8 Gew.-% beträgt. Der Komplex ist ein gelbes, lockeres Pulver, das sich in Wasser schnell zu einer klaren Lösung löst. Die erhaltene Lösung hat eine Konzentration von 0,6-0,8 mg/ml Amphotericin B und kann mit Wasser beziehungsweise physiologischer Kochsalzlösung unbegrenzt verdünnt werden, ohne daß Festsubstanz ausfiele oder die Lösung trübe würde. Die Lösung wurde bei Raumtemperatur 6 Tage lang gelagert und dann spektrophotometrisch untersucht: ihr Wirkstoffgehalt war unverändert.

Beispiel 7

Die vorhergehenden Beispiele beweisen, daß die Polyenantibiotika mit γ-Cyclodextrin Einschlußkomple-

11

xe bilden und dadurch ihre Stabilität und Wasserlöslichkeit bedeutend steigt. Entscheidend ist jedoch, ob auch die antifungale Wirkung geblieben ist. Um diese Frage zu beantworten, wurde die antifungale Wirkung der auf unterschiedliche Weise hergestellten Komplexe sowohl sofort nach der Herstellung wie auch nach Lagerung unter Bedingungen, unter denen der nicht durch Komplexbildung geschützte Wirkstoff bereits beträchtliche Aktivitätseinbußen erleidet, untersucht.

a) Flavofungin-γ-Cyclodextrin-Komplex

Die antifungale Wirkung der wäßrigen Lösung dieses Komplexes wurde an 6 Spaltpilzen und 13 Fadenpilzen untersucht. Die Ergebnisse sind in der Tabelle 7 zusammengefaßt. Auf Flavofungin bezogen war die minimale Hemmkonzentration in jedem Falle 10-25 μg/ml, was der Wirksamkeit des nicht komplexgebundenen Wirkstoffes entspricht. Es ist besonders bemerkenswert, daß die Substanz auch gegen phytopathogene Fusarien wirkt, d.h. eine Anwendung gegen pilzliche Pflanzenschädlinge begründet ist. Der komplexgebundene Wirkstoff wirkt in der gleichen Konzentration wie Flavofungin. Die Untersuchungenwurden mit der Agardiffusionsmethode vorgenommen. Der Komplex hat den Vorteil, daß zum Bereiten der Lösungen keine organischen Lösungsmittel (DMF bzw. DMSO) erforderlich sind. Diese Lösungsmittel wirken nämlich hemmend auf manche Pilze, deshalb muß bei Messungen die Lösungsmittelkonzentration durch Verdünnen mit Wasser auf einen bereits nicht mehr hemmend wirkenden Wert vermindert werden. In der Praxis ist zum Beispiel eine DMSO-Konzentration von unter 1 % zulässig. Manche Polyene neigen jedoch bei derartiger Verdünnung zum Ausfallen, d.h. die Bereitung der zu den Untersuchungen erforderlichen Lösungen stößt oft auf Hindernisse. Empfohlen wird, die Substanz zu pulverisieren und die Bereitung der Lösung sowie deren Verdünnen mit Wasser immer unmittelbar vor der Messung vorzunehmen (J. Ryley, Experimental Approaches to Antifungal Chemotherapy, in: Advances in Pharm. and Chemotherapy, No. 18, Academic Press, London, 1981).

Tabelle 7

Minimale Hemmkonzentrationen (MIC) von Flavofungin-$\beta$-CD-Komplex in vitro

| Testorganismus | MIC ($\mu$g/ml) |
|---|---|
| **1. Hefen und hefeartige Pilze** | |
| Cryptococc. neoform. 78/K-16 | 5-10 |
| Sacchar. cerevisiae OKI 1282 | 10 |
| Candida albicans CBS 562 | 10 |
| Candida tropical CBS 430 | 25 |
| Candida crusei 79/K 47 | 10-25 |
| Candida pseud. tr. | 5-10 |
| **2. Pilze** | |
| Asperg. niger CBS 12648 | 25 |
| Asperg. fumigatus CBS 11326 | 25 |
| Penicillium digitatum CBS 31948 | 25 |
| Penicillium chrysogen. CBS 19646 | 25-50 |
| **3. Pathogene Pilze** | |
| Sporotr. Schenkii CBS 34035 | 25 |
| Trichoph. rubrum CBS 30338 | 25 |
| Trichoph. mentagroph CBS 50148 | 25 |
| Trichoph. (Keratomyc ajelloi OKI) | 25 |
| Epiderm. floccosum OKI, IV. | 25 |
| Microsp. gypseum CBS 10064 | 10-25 |
| Microsp. persic. OKI | 25 |
| **4. Pflanzenpathogene Pilze** | |
| Fusarium moniliform DSM-IMB 11778 | 10-25 |
| Fusarium oxysporum DSM-IMB 10975 | 10-25 |

Die Konzentration des Flavofungins betrug in jedem Falle 10-25 $\mu$g/ml. Die bei der Lösungsbereitung aus Flavofungin auftretenden Probleme sind bei Verwendung des Komplexes gegenstandslos.

b) Nystatin-$\gamma$-CD-Komplex

Die biologische Wertmessung wurde mit der Agardiffusionsmethode unter Verwendung von Candida albicans als Testorganismus vorgenommen. Die Komplexe wurden lediglich in Wasser gelöst, das Nystatin hingegen in calciumchloridhaltigem Methanol, und diese Lösungen wurden dann mit Wasser weiter verdünnt (Ph. Hg. VI. Tomus.II, S. 827).

## Tabelle 8

### Spektrophotometrisch gemessener Wirkstoffgehalt und biologische Aktivität einiger Komplexe

| Komplex | Wirkstoffgehalt (UV-spektrophotometr.) | biol. Aktivität E/mg |
|---------|-----------------------------------------|-----------------------|
| 1 | 7,5 % | 351 |
| 2 | 7,6 % | 362 |
| 3 | 8,0 % | 399 |
| 4 | 8,4 % | 450 |

Die zur Komplexbildung verwendete Nystatingrundsubstanz hatte eine biologische Aktivität von 5033 E/mg.

c) Komplex aus Amphotericin B und γ-CD

Die antifugale Wirksamkeit dieses Komplexes wurde mit der Agardiffusionsmethode und Candida albicans als Testorganismus untersucht. Bezogen auf Amphotericin B war die minimale Hemmkonzentration geringer als 5 μg/ml, das entspricht der Wirksamkeit des nicht komplexgebundenen Wirkstoffes.

Beispiel 8

Untersuchung der Resorption von Polyenantibiotikum-Cyclodextrin-Komplexen

a) Die Resorption von Nystatin aus per os verabreichtem Nystatin-γ-CD-Komplex

Die Resorption des Wirkstoffes wurde an Mäusen, an einem durch intravenöse Applikation einer Suspension von Candida albicans hervorgerufenen Sepsismodell untersucht. Die Sepsis verursachte innerhalb von 2-3 Tagen 100 %ige Mortalität. Den Versuchstieren wurde per os gemäß Beispiel 3 hergestellte, 8 % Nystatin enthaltende Komplex verabreicht. In einem Parallelversuch wurde Nystatin intravenös appliziert. Für beide Gruppen wurde die auf die unbehandelte Kontrolle bezogene Verlängerung der Überlebensdauer ermittelt.

## Tabelle 9

| Nystatin i.v. mg/kg | Verlängerung der Überlebensdauer, % | Nystatin p.o. mg/kg | Verlängerung der Überlebensdauer, % | resorbiertes Nystatin i.v./p.o.Dosis x 100 (%) |
|------|------|------|------|------|
| 0,19 | 30,2 | 3,12 | 33,2 | 6,09 |
| 0,39 | 43,5 | 6,25 | 43,5 | 6,24 |
| 0,78 | 62,7 | 12,5 | 63,0 | 6,24 |
| 1,56 | 34,6 | 25 | 38,9 | 6,24 |

Wenn man diejenigen in Form des Komplexes per os verabreichten Nystatindosen mit denjenigen i.v. applizierten Nystatindosen vergleicht, die eine etwa gleiche Verlängerung der Überlebensdauer bewirkten (Tabelle 9, letzte Spalte),so läßt sich errechnen, daß aus dem Komplex durchschnittlich 6,2 % des Wirkstoffes in die Blutbahn gelangten. Bei den höchsten Dosen (i.v. 1,56 mg/kg, p.o. 25 mg/kg) ist das Zurückfallen der Überlebensdauer bereits auf die schwache Toxizität des Nystatins zurückzuführen.

b) Die Resorption von Nystatin aus subcutan appliziertem Nystatin-γ-CD-Komplex

Weibliche CFY-Ratten eines durchschnittlichen Gewichtes von 180 g wurden mit Nystatin, mit

Nystatin-$\gamma$-CD-Komplex (Wirkstoffgehalt9 8 %) beziehungsweise mit $\gamma$-CD behandelt. Dazu wurde aus den zu untersuchenden Substanzen mit einer Tween 80 enthaltenden physiologischen Kochsalzlösung eine Suspension bereitet und diese subcutan appliziert. Der Zusammenhang zwischen den Dosen und der Mortalität ist aus der Tabelle 10 ersichtlich.

Während im Falle des Komplexes bei einer Dosis von 20 mg/kg 90 % der behandelten Tiere innerhalb von 48 Stunden verendeten, ging von den mit freiem Nystatin behandelten Tieren auch bei einer Dosis von 150 mg/kg innerhalb des Beobachtungszeitraumes von einer Woche kein einziges ein. Zur Kontrolle wurde 10 Tieren subcutan so viel $\gamma$-CD appliziert, wie die zu 90 % verendete Gruppe zusammen mit dem Komplex erhielt (302 mg/kg). Innerhalb des einwöchigen Beobachtungszeitraumes verendete kein einziges Tier.

### Tabelle 10

| | Nystatin | | | | | Nystatin-$\gamma$-CD-Komplex | | | | |
|---|---|---|---|---|---|---|---|---|---|---|
| Dosis mg/kg | Anzahl der Tiere unter-sucht | verendet nach | | | Dosis $\mu$g/kg | Anzahl der Tiere unter-sucht | verendet nach | | | |
| | | $24^h$ | $48^h$ | $7^d$ | | | $24^h$ | $48^h$ | $7^d$ (1 Woche |
| 10 | 10 | 0 | 0 | 0 | 10 | 10 | 0 | 0 | 0 | |
| 20 | 10 | 0 | 0 | 0 | 20 | 10 | 8 | 1 | 0 | |
| 100 | 5 | 0 | 0 | 0 | | | | | | |
| 150 | 5 | 0 | 0 | 0 | | | | | | |

Aus der Untersuchung geht eindeutig hervor, daß aus dem Nystatin-$\gamma$-CD-Komplex bei subcutaner Applikation eine toxische Dosis resorbiert wird, während das freie Nystatin bei subcutaner Applikation überhaupt nicht oder nur in vernachlässigbar geringem Maße resorbiert wird.

Qualitativ wurde die Resorption auch durch Messung des Blutspiegels untersucht, weil das resorbierte,in die Blutbahn gelangte Nystatin im UV-Spektrum des Blutserums nachweisbar ist. Von subcutan appliziertem freiem Nystatin war auch im Falle einer Dosis von 200 mg/kg im Blut nichts nachzuweisen, während der Komplex bereits bei einer Dosis von 20 mg/kg einen gut meßbaren Blutspiegel ergab: 2-4 Stunden nach der Verabreichung waren im Blut 6-10 $\mu$g/ml nachweisbar.

Beispiel 9

Verringerung der hämolysierenden Wirkung von Polyenantibiotika durch Komplexbildung mit $\gamma$-CD

a) Nystatin-$\gamma$-CD-Komplex

Die hämolytische Wirkung von Nystatin, Nystatin-$\gamma$-CD-Komplex (Wirkstoffgehalt: 8%) und $\gamma$-CD wurde an aus menschlichem Blut hergestellten Suspensionen roter Blutkörperchen nach der Methode von T. Irie et al. (J. Pharm. Dyn. 6, 408-414 [1983]) untersucht. Die Ergebnisse werden in Form der zu 50 %iger Hämolyse gehörenden Konzentrationen ($EH_{50}$) angegeben. Der für freies Nystatin erhaltene Wert ($EH_{50}$ = 57 $\mu$g/ml) zeigt eine gute Übereinstimmung mit dem aus der Literatur bekannten Wert von $EH_{50}$ = 50 $\mu$g/ml (J. Antibiotics 22, 1080-81 [1979]). Für den Nystatin-$\gamma$-CD-Komplex wurde, berechnet als Wirkstoff, ein Wert von $EH_{50}$ = 115 $\mu$g/ml gefunden, d.h. im Komplex sinkt die hämolysierende Wirkung des Nystatins auf die Hälfte. Bei einer Untersuchung der Konzentrationsabhängigkeit der hämolytischen Wirkung erwies sich, daß der Nystatin-$\gamma$-CD-Komplex bis zu einer Konzentration von 70 $\mu$g/ml Nystatin praktisch nicht hämolytisch wirkt, während freies Nystatin in dieser Konzentration bereits 70 %ige Hämolyse hervorruft. Auch die hämolytische Wirkung einer der im Komplex gebundenen $\gamma$-CD-Menge entsprechenden Menge $\gamma$-Cyclodextrin wurde untersucht. Dabei wurde gefunden, daß $\gamma$-CD in Übereinstimmung mit Angaben aus der Literatur bis zu einer Konzentration von 1600 $\mu$g/ml keine Hämolyse verursacht.

b) Komplex aus Amphotericin und $\gamma$-CD

Die hämolytische Wirkung dieses Komplexes (hergestellt gemäß Beispiel 5, Wirkstoffgehalt 0,6 %) wurde auf die unter a) angegebene Weise untersucht. Für freies Amphotericin B ergab sich ein Wert von $EH_{50}$ = 4,8 $\mu$g/ml, was ebenfalls gut mit den Literaturangaben ($EH_{50}$ = 5 $\mu$g/ml)übereinstimmt. Der

Komplex aus Amphotericin und γ-CD verursachte auch in einer als Wirkstoff berechneten Konzentration von 25 μg/ml nur eine 2,6 %ige Hämolyse, während freies Amphotericin in dieser Konzentration bereits 92,4 %ige Hämolyse hervorruft.

Herstellung von Polyenantibiotikum-γ-Cyclodextrin-Komplexe enthaltenden Arzneimittelpräparaten und Pflanzenschutzmitteln

Beispiel 10

Puder mit 100 000 IE/g Nystatingehalt

250 mg Nystatin-γ-Cyclodextrin-Komplex werden mit Talkum zu 1 g ergänzt (der Komplex hat eine biologische Aktivität von 400 E/mg).

Beispiel 11

Augen- bzw. Ohrentropfen mit 10 000 bzw. 30 000 E/ml Nystatingehalt

30 beziehungsweise 70 mg Nystatin-γ-CD-Komplex, bereitet durch steriles Lyophilisieren eines homogenen Mediums von 400 IE/mg, werden mit Wasser auf 1 ml aufgefüllt. Die Lösung wird zweckmäßig frisch bereitet. Bei Raumtemperatur gelagert ist sie innerhalb von 10 Tagen verwendbar (mehr als 70 % ihrer Aktivität sind auch dann noch erhalten).

(Die im Handel erhältlichen ähnlichen Präparate enthalten das Nystatin in mikronisierter Form. Zur Anwendung muß aus diesem Pulver eine Suspension bereitet werden, die vor Licht geschützt im Kühlschrank zu lagern ist. Trotzdem verliert sie den größten Teil ihrer Aktivität in einer Woche.)

Beispiel 12

Nystatinsalbe mit 100 000 IE/g Nystatingehalt

1 g Nystatin-γ-CD-Komplex (Aktivität 400 IE/mg) wird mit 3 g Unguentum-Simplex-Salbe vermischt.

Beispiel 13
Nystatintabletten mit 200 000 IE/Tablette
500 mg Nystatin-γ-CD-Komplex (400 IE/mg)
145 mg Lactose
30 mg Kartoffelstärke
20 mg Talkum
5 mg Calciumstearat
pro Tablette. Gesamtgewicht einer Tablette: 700 mg Die Tabletten werden in an sich bekannter Weise durch unmittelbares Verpressen hergestellt.

Beispiel 14

Scheidenzäpfchen mit 100 000 IE/Stück

250 mg Nystatin-γ-CD-Komplex einer durchschnittlichen Aktivität von 400 IE/mg werden mit 2 g Adeps solidus (festes Fett) oder Massa polyoxyaetheni (Polyäthylenglycol) enthaltender Suppositorienmasse verarbeitet.

Beispiel 15

Amphotericin B enthaltende Injektionslösung mit 5 beziehungsweise 10 mg/ml Wirkstoffgehalt, zur Anwendung als Infusion

700 beziehungsweise 1400 mg eines 0,8 % Wirkstoff enthaltenden, unter sterilen Bedingungen lyophilisierten Komplexes aus Amphotericin und γ-CD werden in Pulverampullen gefüllt. Zum Auflösen des Präparates sind 10 beziehungsweise 20 ml steriles destilliertes Wasser notwendig. Zur Verwendung als Infusion kann die Lösung mit isotonischer Salzlösung oder mit 5 %iger Dextroselösung unbeschränkt verdünnt werden, die Gefahr der Niederschlagsbildung besteht nicht.

Beispiel 16

Nystatinlösung für Gewebekulturen zum Vermeiden von Pilzbefall (10 000 E/ml)

25 mg lyophilisierter Nystatin-γ-CD-Komplex der Aktivität von 400 E/mg werden in 1 ml destilliertem Wasser gelöst. Die auf diese Weise bereitete Lösung zeigte nach 10 Tagen Lagerung bei Raumtemperatur, vor Licht nicht geschützt, noch eine Aktivität von 7300 E/ml. In Gewebekulturen wird anhängend von der Art der Kultur im allgemeinen eine Konzentration von 10-500 E/ml angewendet. Diese Konzentration wird durch entsprechendes Verdünnen der Lösung eingestellt.

Beispiel 17

Amphotericin B enthaltende Lösung für Gewebekulturen zum Vermeiden von Pilzbefall (250 mg/ml)

630 mg eines 0,8 %, Wirkstoff enthaltenden Komplexes aus Amphotericin B und γ-CD werden vor der Anwendung mit destilliertem Wasser zu 20 ml Lösung gelöst. Abhängend von der Art der Gewebekultur beträgt die angewendete Konzentration 5-50 μg/ml und wird durch Verdünnen mit Wasser eingestellt.

Beispiel 18

Saatgutbeizmittel

Nystatin als Fungizid mit breitem Wirkungsspektrum ergänzt das Wirkungsspektrum von Benomyl und Carbendazim in glücklicher Weise. Die günstige Summenwirkung ließ sich auch durch Freilandversuche bestätigen. Es wurde jedoch bisher wegen seiner hochgradigen Wasserunlöslichkeit und seiner Instabilität in wäßrigen Systemen für diesen Zweck nicht eingesetzt.

Für Saatgutbeizen empfohlene Zusammensetzung:

25 % Nystatin-γ-CD-Komplex gemäß Beispiel 4 (10 % Wirkstoffgehalt, Aktivität: 450 E/mg)
40 % Benomyl
35 % Träger- und Hilfsstoffe.

Dieses Präparat wird in an sich bekannter Weise hergestellt. Auf 100 g Saatgut rechnet man vorzugsweise etwa 200 g des Präparates.

**Patentansprüche**

1. Mit γ-Cyclodextrin gebildete Komplexe von Polyenantibiotika.

2. Flavofungin-γ-Cyclodextrin-Komplex.

3. Nystatin-γ-Cyclodextrin-Komplex.

4. Amphotericin-B-γ-Cyclodextrin-Komplex.

5. Verfahren zur Herstellung wasserlöslicher und stabiler Polyenantibiotikum-Derivate, dadurch gekennzeichnet, daß man in Wasser begrenzt lösliche beziehungsweise praktisch unlösliche Polyenantibiotika unter Rühren in Gegenwart von Wasser mit γ-Cyclodextrin umsetzt.

6. Verfahren nach Anspruch 5, dadurch gekennzeichnet, daß man als Antibiotikum Flavofungin, Amphotericin B, N statin, Candicidin oder Pimaricin verwendet.

7. Verfahren nach Anspruch 5 oder 6, dadurch gekennzeichnet, daß man die Polyenantibiotika mit 1-40 mMol γ-Cyclodextrin auf 1 mMol Antibiotikum gerechnet in Gegenwart von 1-500 ml, vorzugsweise 20-500 ml Wasser, vorzugsweise bei 25-55 °C unter Rühren, umsetzt und den erhaltenen Antibiotikum-γ-Cyclodextrin-Komplex gewünschtenfalls isoliert.

8. Verfahren nach eine der Ansprüche 5-7, dadurch gekennzeichnet, daß man die Polyenantibiotika mit 1-40 mMol γ-Cyclodextrin auf 1 mMol Antibiotikum gerechnet in 1-50 ml eines 20-80 vol.-%igen, wassermischbaren Lösungsmittels, vorzugsweise Äthanol, unter Rühren beziehungsweise Kneten umsetzt.

9. Verfahren nach einem der Ansprüche 5-8, dadurch gekennzeichnet, daß man den erhaltenen Antibiotikum-γ-Cyclodextrin-Komplex durch Lyophilisieren, Zerstäubungstrocknung, Vakuumeindampfen oder Vakuumtrocknung bei niedriger Temperatur isoliert.

10. Arzneimittelpräparat mit antifungalar Wirkung, gekennzeichnet durch einen Gehalt an 1-80 Gew.-% Polyenantibiotikum-γ-Cyclodextrin-Komplex.

11. Pflanzenschutzmittel mit antifungaler Wirkung, gekennzeichnet durch einen Gehalt an 0,25-30 Gew.-% Polyenantibiotikum-γ-Cyclodextrin-Komplex.

12. Zusatzsstoff mit antifungaler Wirkung für Gewebekulturen zur Vermeidung von Pilzbefall, gekennzeichnet durch einen Gehalt an 0,02-5 mg/ml Polyenantibiotikum-γ-Cyclodextrin-Komplex.

## Claims

1. Complexes of polyene antibiotics formed with γ-cyclodextrin.

2. Flavofungin-γ-cyclodextrin complex.

3. Nystatin-γ-cyclodextrin complex.

4. Amphotericin B-γ-cyclodextrin complex.

5. Process for the preparation of water-soluble and stable polyene antibiotic derivatives, characterised in that polyene antibiotics which have limited solubility or are virtually insoluble in water are reacted with γ-cyclodextrin with stirring in the presence of water.

6. Process according to Claim 5, characterised in that flavofungin, amphotericin B, nystatin, candicidin or pimaricin is used as the antibiotic.

7. Process according to Claim 5 or 6, characterised in that the polyene antibiotics are reacted with 1-40 mmol of γ-cyclodextrin per mmol of antibiotic in the presence of 1-500 ml, preferably 20-500 ml, of water, preferably at 25-55°C, with stirring, and the antibiotic-γ-cyclodextrin complex obtained is isolated if desired.

8. Process according to one of Claims 5-7, characterised in that the polyene antibiotics are reacted with 1-40 mmol of γ-cyclodextrin per mmol of antibiotic in 1-50 ml of a 20-80% strength by volume, water-miscible solvent, preferably ethanol, with stirring or kneading.

9. Process according to one of Claims 5-8, characterised in that the antibiotic-γ-cyclodextrin complex obtained is isolated by lyophilisation, spray drying, vacuum evaporation or vacuum drying at low temperature.

10. Medicament preparation having antifungal activity, characterised in that it contains 1-80% by weight of polyene antibiotic-γ-cyclodextrin complex.

11. Plant protection agent having antifungal activity, characterised in that it contains 0.25-30% by weight of polyene antibiotic-γ-cyclodextrin complex.

12. Additive having antifungal activity for tissue cultures for avoiding fungal attack, characterised in that it contains 0.02-5 mg/ml of polyene antibiotic-γ-cyclodextrin complex.

## Revendications

1. Complexes de polyène-antibiotiques formés avec la γ-cyclo-dextrine.

2. Complexe flavofungine-γ-cyclodextrine.

3. Complexe nystatine-γ-cyclo-dextrine.

4. Complexe amphotéricine-B-γ-cyclodextrine.

5. Procédé de préparation de dérivés polyèneantibiotiques stables et hydrosolubles, caractérisés en ce qu'on fait réagir sous agitation en présence d'eau avec de la γ-cyclo-dextrine des polyène-antibiotiques à solubilité limitée dans l'eau ou pratiquement insolubles dans l'eau.

6. Procédé selon la revendication 5, caractérisé en ce qu'on utilise comme antibiotique de la flavofungine, de l'amphotéricine B , de la Nystatine, de la candicidine ou de la pimaricine.

7. Procédé selon la revendication 5 ou 6, caractérisé en ce qu'on fait réagir les polyène-antibiotiques avec 1-40 mmoles de γ-cyclodextrine, calculé pour 1 mmole d'antibiotique, en présence de 1-500 ml, de préférence 20-500 ml d'eau, de préférence entre 25 et 55° C sous agitation, et si c'est souhaité on isole le complexe obtenu antibiotique-γ-cyclodextrine.

8. Procédé selon l'une des revendications 5-7, caractérisé en ce qu'on fait réagir les polyène-antibiotiques avec 1-40 mmoles de γ-cyclodextrine, calculé pour 1 mmole d'antibiotique, dans 1-50 ml d'un solvant miscible à l'eau à 20-80% en volume, de préférence de l'éthanol, sous agitation ou malaxage.

9. Procédé selon l'une des revendications 5-8, caractérisé en ce qu'on isole le complexe obtenu antibiotique-γ-cyclodextrine par lyophilisation, atomisation, évaporation sous vide ou séchage sous vide à basse température.

10. Préparation de médicaments à action antifungique, caractérisée par une teneur de 1-80% en poids de complexe polyène-antibiotique-γ-cyclodextrine.

11. Agent phytosanitaire à effet antifungique, caractérisé par une teneur de 0,25-30% en poids de complexe polyène-antibiotique-γ-cyclodextrine.

12. Additif à effet antifungique pour les cultures de tissus afin d'éviter la contamination par des champignons, caractérisé par une teneur de 0,02-5 mg/ml de complexe polyène-antibiotique-γ-cyclodextrine.